Europäisches Patentamt

European Patent Office

Office européen des brevets

(19)

(11) **EP 0 118 240 B2**

(12) **NEW EUROPEAN PATENT SPECIFICATION**

(45) Date of publication and mention
of the opposition decision:
**18.06.1997 Bulletin 1997/25**

(45) Mention of the grant of the patent:
**26.07.1989 Bulletin 1989/30**

(21) Application number: **84300940.8**

(22) Date of filing: **14.02.1984**

(51) Int Cl.6: **C08L 3/00**, A61K 9/48

(54) **Process for injection moulding starch**

Spritzgussverfahren zur Formgebung von Stärke

Procédure pour moulage par injection d'amidon

(84) Designated Contracting States:
**AT BE CH DE FR GB IT LI LU NL SE**

(30) Priority: **18.02.1983 US 467982**
**13.02.1984 US 579318**

(43) Date of publication of application:
**12.09.1984 Bulletin 1984/37**

(73) Proprietor: **WARNER-LAMBERT COMPANY**
**Morris Plains New Jersey 07950 (US)**

(72) Inventors:
• **Wittwer, Fritz**
**CH-4411 Lupsingen (CH)**
• **Tomka, Ivan**
**CH-1722 Bourguillon (CH)**

(74) Representative: **Mercer, Christopher Paul et al**
**Carpmaels & Ransford**
**43, Bloomsbury Square**
**London WC1A 2RA (GB)**

(56) References cited:
BE-A- 654 605          DE-A- 1 965 584
DE-B-12 295 584        GB-A- 2 050 459
JP-A- 16 645           US-A- 2 788 546
US-A- 2 805 966        US-A- 3 117 014
US-A- 3 137 592        US-A- 3 265 509
US-A- 3 265 510        US-A- 4 026 986
US-A- 4 076 846        US-A- 4 218 350
US-A- 5 275 774

• CHEMICAL ABSTRACTS, vol. 87, no. 22, 28th
  November 1977, page 314, no. 172903j,
  Columbus, Ohio, US; & JP - A - 77 79 019
• Cereal Chemistry, vol 52(3), 1975, pp 283-297
• Cereal Chemistry, vol. 54(3), 1977, pp 436-443
• Cereal Chemistry, vol. 57(1), 1980, pp 4-9
• Die Stärke, vol. 23(19, 1971, pp 8-11
• Die Stärke, vol. 29(2), 1977, pp 48-52
• Die Stärke, vol. 45(8), 1993, pp 276-280
• Starch: Chemistry and Technology (ed Whistler
  et al), 1st ed., 1967, pp 513-515
• Starch: Chemistry and technology (ed Whistler
  et al), 2nd ed., 1984, pp 183-247
• Encyclopedia of Chemical Technology
  (Kirk-Othmer), 2nd ed., vol. 15, pp 790-811
• Encyclopedia of Chemical Technology
  (Kirk-Othmer), 2nd ed., vol. 18, pp 195-197
• Extrusion of Foods (CRC Press), 1981, pp 45-52
• Cereal Science Today, vol. 18(9), Abstract 57
• Chemistry and Industry of Starch (ed Kerr), 1950,
  pp 250-254
• Grundlagen der Phys. Chemie, 7. Auflage, 1968,
  Seiten 855-856
• Food Processing Engineering, vol. 1, 1980, pp
  195-197

**Description**

The present invention relates to a moldable starch composition for use in an injection molding device to produce capsules. The present invention utilizes starch made from corn, wheat, potatoes, rice, tapioca and the like. Said types of starch have a usual molecular mass range of 10,000 to 20,000,000 Dalton.

The starch contains about 0 to 100% amylose, and about 100 to 0% of amylo-pectin; preferably 0 to 70% of amylose, and about 95 to 10% of amylo-pectin, and is most preferably potato starch and corn starch.

When in the following description the term "starch" is used, this refers to starch which is not chemically modified.

It is primary object of the present invention to utilize starch compositions in the production of injection molded products, especially capsules.

Concurrently with this application please also refer to European Published Patent Application No. 0 090 600 filed 24th March, 1983.

Capsule-making machines have been developed to utilize dip-molding technology. Such technology involves the dipping of capsule-shaped pins into a gelatin solution, removing the pins from the solution, drying of the gelatin upon the pins, stripping off the gelatin capsule parts from the pins, adjusting for length, cutting, joining and ejecting the capsules. Prior art capsule-making machines have utilized the combination of mechanical and pneumatic elements to perform these functions at speeds up to about 1,200 size 0 capsules per minute. While the above described apparatuses are in general suitable for the intended purposes, it is desirable to produce capsules by injection molding at considerably higher speed, while at the same time precisely controlling the properties of the starch in order to produce the capsules hygenically and with minimum dimensional deviations so that the capsules can be filled on high speed equipment.

A prerequisite for any material to be moldable by an injection process is its ability to pass a glass transition point at a temperature compatible with the thermal stability of the material and the technical possibilities of an injection molding device. A pre-requisite of any material to deliver shaped products of high dimensional stability in an injection molding process is its minimum elastic recovery after the mold is opened. This can be achieved by setting the dispersity of said material at the molecular level during the injection process.

*Shirai* et al. in US Patent 4,216,240 describes an injection molding process to produce an oriented fibrous protein product. The fibrous product obtained by this process differs fundamentally from the transparent glasslike material of the capsules obtained from the present invention. Furthermore to obtain a flowable mass for the molding process, the protein mixtures used by Shirai et al. have to be denatured and thus lose their capacity to undergo dissolution.

*Nakatsuka* et al. in US Patent 4,076,846 discloses binary mixtures of starch with protein material or alkali metal or alkaline earth metal salts of protein to obtain an edible shaped article by an injection molding process. With the present invention shaped articles can be produced with starch without admixture of protein materials therewith. The disclaimer added in part (a) of present claims 1, 18 and 19 and in claim 17 excludes the disclosure of Nakatsuka from the present invention.

*Heusdens* et al. in US Patent No. 3,911,159 discloses the formation of filamentous protein structures to obtain edible products of improved tenderness. With the present invention shaped articles are produced without a filamentous protein structure.

In the Belgian Patent No. 654,605 the production of flexible films from materials with a high amylose content is described using extrusion techniques.

The use of an injection molding device for producing capsules with chemically non-modified starch is new and has not been suggested in the technical literature. Many useful products can be prepared by the injection molding of starch other than capsules with the necessity of high form stability and minimum dimensional deviations. These products would include candies, packaging containers for food-stuffs, pharmaceuticals, chemicals, dyestuffs, spices, fertilizing combinations, seeds, cosmetics and agricultural products and matrices of various shapes and size of starch compositions containing substances and/or active ingredients including food stuffs, pharmaceuticals, chemicals, dyestuffs, spices, fertilizing combinations, seeds, cosmetics and agricultural products, which are micro-dispersed within the matrix and released from it through disintegration and/or dissolution and/or bioerrosion and/or diffusion depending on the solubility characteristics of the used starch composition. Some of these products may also result in a controlled release delivery system for the enclosed substance. Furthermore, medical and surgery products can be prepared by injection molding starch compositions. The biodegradable nature of starch makes it environmentally desirable over certain materials presently being used. In addition, the non-toxic nature of the materials further enhances their desirability as a material to be used in the injection molding industry. It is an object of this invention to encompass all injection molded products that may be produced by the teachings of the present invention. The present invention distingushes from the known prior art described above, by the recognition that starch possesses a dissolution point within a temperature range usable for an injection molding process, provided the water content of the starch lies within a characteristic range, giving allowance to avoid any essential drying or humidification processes of the capsules. Above the dissolution point the starch is in the state of molecular dispersity. Due to the present invention the starch during the injection molding process is for a considerable time at a temperature which is higher than the temperature of the dissolution point. When materials, such as medicaments and food-stuffs, are dispersed in the starch compositions, quantities can not be employed that will so effect the properties of the starch that it will no longer be injection moldable.

According to a first aspect of the present invention, there is provided a process for forming starch into an article using an injection molding technique, which process is defined in claim 1.

Preferred features of the process are defined in claims 2 to 12.

The present invention also provides an injection molded article, in particular a capsule, or a product whenever produced by a process as defined in any one of claims 1 to 12.

According to a second aspect of the present invention, there is provided the use of a starch composition, as defined in claim 17.

The present invention further provides, in a third aspect, a composition in the form of a molecular dispersion of starch and water, as defined in claim 17, and a moulded article made from the composition, as defined in claim 20.

The present invention yet further provides a process for preparing a composition in the form of a molecular dispersion of starch and water, as defined in claim 19.

The invention both as its organization and method of operation together with further objects and advantages thereof will best be understood by reference to the following specification and taken in conjunction with the accompanying drawings.

Fig. 1 is a schematic layout of a reciprocating screw injection molding device for making capsule parts;

Fig. 2 is a schematic of an injection molding work cycle for making capsule parts;

Fig. 3 is a schematic of an embodiment of a combined injection molding device-microprocessor apparatus for capsule parts;

Fig. 4 is an expanded schematic of the exit end of the injection molding device;

Fig. 5 is the diagram of dependence of shear viscosity of starch within the pertinent ranges of the shear rate in the present invention;

Fig. 6 is the diagram of molding area for starch within the ranges of temperature and pressure of starch for the present invention;

Fig. 7 is the diagram of dependence of glass transition temperature range and melting temperature range for the pertinent water content ranges of starch;

Fig. 8 is the diagram of dependence of differential calorimeter scan in which the heat consumption rate of the starch is plotted for the pertinent temperature range of the present invention; and

Fig. 9 is a diagram of dependence of equilibrium water content of the starch in the water activity program.

Detailed description of the preferred embodiment

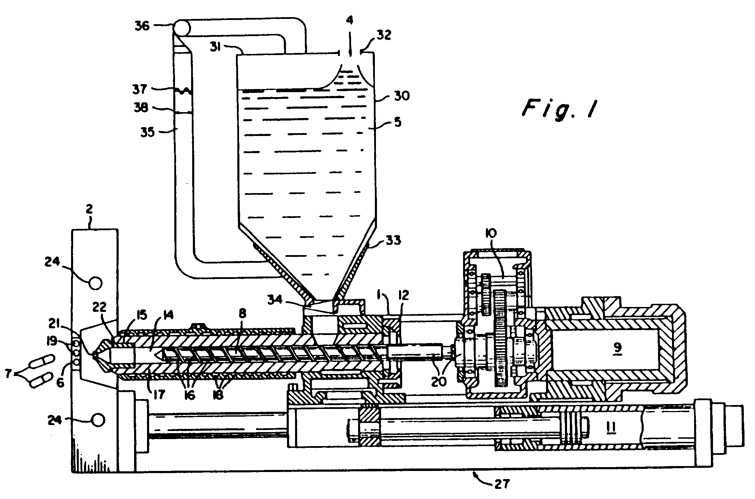

Referring now to Fig. 1 the injection molding device 27 generally consists of three units: a hopper unit 5, an injection unit 1 and a molding unit 2.

The function of the hopper unit 5 is receiving, storing, maintaining and feeding starch 4 at a constant temperature and at a constant water content. The hopper unit 5 comprises a vertical cylinder 30 having a closed top 31 with an inlet 32 therein to receive starch 4. At the bottom of the vertical cylinder 30 is a closed conical funnel 33 and a discharge outlet 34 to feed starch 4 into an inlet 34 of the injection unit 1. There is an air duct 35 communicating between the closed top 31 and the conical funnel 33 wherein air is circulated by a blower 36, the air temperature is maintained by a thyristor 37 and the air relative humidity is maintained by a steam injector 38.

The function of the injection unit 1 is melting, dissolving in water, and plasticizing in the extruder barrel 17 the starch 4 fed from the hopper unit 5 into the extruder inlet 54 and injecting the plasticized starch 14 into the molding unit 2.

The function of the molding unit 2 is automatically holding, opening and closing the mold 6 having capsule shaped cavities 19 therein, and ejecting the capsule parts 7 therefrom.

Within the injection unit 1 the screw 8 both rotates and undergoes axial reciprocal motion. When the screw 8 rotates, it performs the functions of melting, dissolving in water, and plasticizing the starch 4. When the screw 8 moves axially, it performs the function of injecting by transporting and remming the plasticized starch 14 into the mold 6. The screw 8 is rotated by a variable-speed hydraulic motor 9 and drive 10, and its axial motion is reciprocated by a duplex hydraulic cylinder 11.

Compression of the plasticized starch 14 in front of the rotating screw 8 forces back the screw assembly 20 containing the screw 8, the drive 10 and the motor 9. When the screw assembly 20 reaches a preset back position a limit switch 12 is contacted. When a defined time has elapsed during which the starch 4 becomes fully plasticized starch 14 the hydraulic cylinder 11 brings the screw assembly 20 forward and uses the screw 8 as a ram for the plasticized starch 14 to be injected through a valve body assembly 50 (Fig. 4) including a one-way valve 15, a needle valve 23, nozzle 22 and an outlet port 21 into the molding unit 2. The one-way valve 15 prevents the plasticized starch 14 from going back over the helical flutes 16 of the screw 8. The extruder barrel 17 has heating coils 18 to heat the starch 4 while it is being compressed by the screw 8 into plasticized starch 14. It is desirable for the plasticized starch 14 to be heated at the lowest possible temperature and to be transported with the lowest possible speed of the screw 8. The speed of the screw 8 and the heating of the plasticized starch 14 within the extruder barrel 17 by the steam heating coils 18 control the quality and the output rate of the plasticized starch 14 injected into the molding unit 2. The molding unit 2 holds the mold 6 having capsule shaped cavities 19 into which the plasticized starch 14 is injected and maintained under pressure. Refrigerant cooling conduits 24 encircle the mold 6 so that when the plasticized starch 14 in the mold 6 has cooled and sufficiently solidified, the molding unit 2 opens, the mold 6 separates and the capsule parts 7 are ejected.

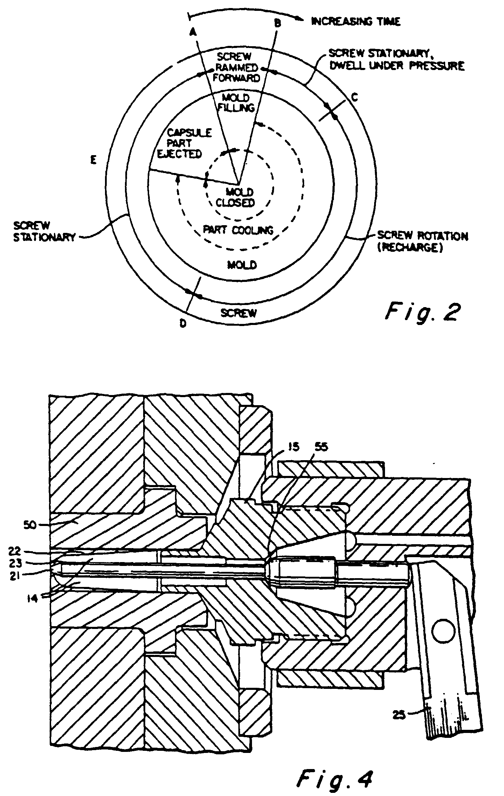

Referring now to Fig. 1 and also to Fig. 2 which depicts the injection molding work cycle for starch 4 containing approximately 20% water, by weight. In general the work cycle of starch 4 is as follows in the injection molding device 27 of the present invention:

a. starch 4 is fed into the hopper unit 5 where it is received, stored and maintained under conditions of temperature ranging from ambient to 100°C, pressure ranging from 1-5×10$^5$ Newtons per square meter (N×m$^{-2}$) and water content ranging from 5 to 30% by weight of starch,

b. the stored starch 4 is melted under controlled condition of temperature ranging from 80 to 240°C, water content ranging from 5 to 30% by weight of starch and pressure ranging from 600 to 3000×10$^5$ N×m$^{-2}$,

c. the molten starch 4 is dissolved in water under controlled conditions of temperature ranging from 80 to 240°C pressures ranging from 600 to 3000×10$^5$ N×m$^{-2}$ and water content ranging from 5 to 30% by weight of starch,

d. the dissolved starch 4 is plasticized under controlled conditions of temperature ranging from 80 to 240°C, pressure ranging from 600 to 3000×10$^5$ N×m$^{-2}$ and water content ranging from 5 to 30% by weight of starch,

e. the plasticized starch 14 is injected into the mold 6 under controlled conditions of temperature above 80°C, injection pressure ranging from 600 to 3000×10$^5$ N×m$^{-2}$ and a clamping force of the mold 6 with a range of approximately 100 to 10,000 Kilo Newton, and

f. the capsule-shaped parts 7 are ejected from the plasticized starch 14 within the mold 6.

Beginning at point A of Fig. 2 the screw 8 moves forward and fills the mold 6 with plasticized starch 14 until Point B and maintains the injected plasticized starch 14 under high pressure, during what is called the hold time from point B until Point C of Fig. 2. At Point A the one-way valve 15 at the end of the screw 8 prevents the plasticized starch 14 from flowing back from the cylindrical space in front of the screw 8 into the helical flutes of screw 8. During hold time, additional plasticized starch 14 is injected, off-setting contraction due to cooling and solidification of the plasticized starch 14. Later. the outlet port 21, which is a narrow entrance to the molding unit 2 closes, thus isolating the molding unit 2 from the injection unit 1. The plasticized starch 14 within the mold 6 is still at high pressure. As the plasticized starch 14 cools and solidifies, pressure drops to a level that is high enough to ensure the absence of sinkmarks, but not so high that it becomes difficult to remove the capsule parts 7 from the capsule-shaped cavities 19 within the mold 6. After the outlet port 21 closes, at Point C, screw 8 rotation commences. The plasticized starch 14 is accommodated in the increased cylindrical space in front of the screw 8 created by its backward axial motion until Point D. The flow rate of the plasticized starch 14 is controlled by the speed of the screw 8 and the pressure is controlled by the back pressure (i.e., the hydraulic pressure exerted on the screw assembly 20) which in turn determines the pressure in the plasticized starch 14 in front of the screw 8. After plasticized starch 14 generation for the next shot into the mold 6, the screw 8 rotation ceases at Point D. The starch 4 on the stationary screw 8 is held at melt temperature from Points D to E by heat conduction from the heating coils 18 on the extruder barrel 17. Meanwhile, the solidified capsule parts 7 are ejected from the mold 6. Thereafter, the mold 6 closes to accept the next shot of plasticized starch 14. All of these operations are automated and controlled by a microprocessor as hereinafter described.

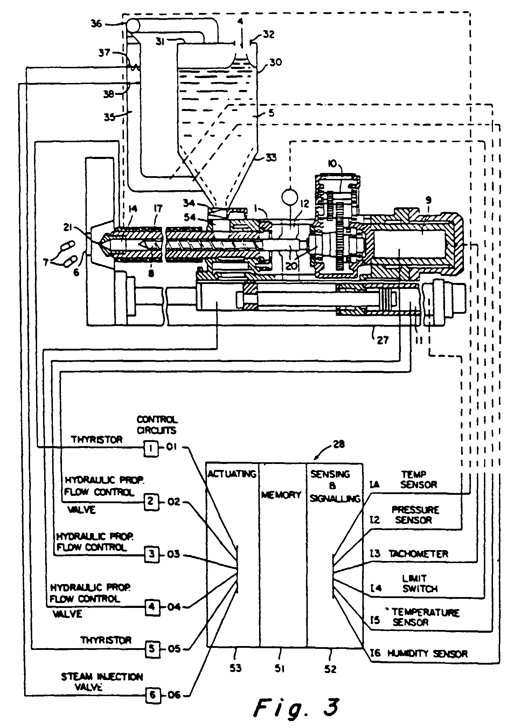

Referring now to Fig. 2 and also to Fig. 3. The injection molding work cycle of Fig. 2 is accomplished on the injection molding device 27 of Fig. 3 by hydraulic and electrical components and the corresponding circuits controlled by the microprocessor 28 of Fig. 3.

Through the use of solid-state circuitry and of speed, temperature, limit and pressure switches for the electric and hydraulic systems, the microprocessor 28 of the present invention utilizes command signals in its memory 51 for the parameters of time, temperature and pressure conditions of Table 1 below for the injection molding work cycle of Fig. 2 to be accomplished by the injection molding device of Fig. 3 for producing capsule parts 7.

**Ranges of time, temperature and pressure at the top of the screw for the injection molding cycle of Fig. 2**

## POINTS

EP 0 118 240 B2

| | A | B | C | D | E |
|---|---|---|---|---|---|
| Time | $10^{-2}$-1 | $10^{-2}$-1 | $10^{-2}$-1 | $10^{-2}$-1 | $10^{-2}$-1 |
| Temperature (° Celsius) | ambient - 100 | 80 - 240 | 80 - 190 | 80 - 240 | 80 - 240 |
| Pressure ($10^{5}$ x N x m$^{-2}$) | | A-B 600 - 3000 | B-C 600 - 3000 | C-D 10 - 1000 | D-E 10 - 1000 |

Referring now to Fig. 3 illustrating the combined injection molding device 27 and microprocessor 28 utilizing the method of the present invention.

The combined injection molding device 27 and microprocessor 28 comprises six control circuits of which five are closed-loop, fully analog, and one is on-off. Starting at molding cycle Point A in Fig. 2, the injection molding work cycle operates as follows:

When sufficient plasticized starch 14 has accumulated in front of the screw 8 (microprocessor limit switch controlled) and also when the screw assembly 20 carrying the screw 8, drive 9 and hydraulic motor 11 has been pushed far enough backwards against a constant back-pressure as controlled by control circuit 2, limit switch 12 will be actuated by position sensing circuit $I4$. The two conditions for actuating cylinder 11 (barrel unit forward) are: 1) clamping force of the mold is built-up, and 2) limit switch 12 is activated. This rams the barrel 17 together with the nozzle 14 with screw assembly 20 forward, thus for sealing purposes. Sufficient pressure is controlled by control circuit 2 with means of pressure sensor $I_2$. Under these conditions hydraulic piston 9 rams the screw assembly 20 forward, thus injecting the plasticized starch 14 into the mold 6 when molding cycle Point B of Fig. 2 is reached, and, as controlled by the microprocessor 28, the screw 8 remains for a certain period of time until Point C stationary in this forward position under high pressure.

From molding cycle Point B of Fig. 2 onwards the plasticized starch 14 cools down in the mold 6 and the port 21 closes at molding cycle Point C of Fig. 2.

At molding cycle Point C of Fig. 2 the screw 8 starts to rotate again and the hydraulic pressure reduces from holding pressure to back pressure in the hydraulic cylinder 11. This pressure is set less than the holding pressure at Point C.

The barrel 17 is kept under constant pressure towards the mold 6 by the pressure in the back position of the hydraulic cylinder 11. This is achieved by means of the control circuit 2 where a proportional hydraulic valve is controlled by a pressure sensor circuit $I_2$.

As the screw 8 rotates a recharge of starch 4 is made from the hopper 5. During a certain time period and at a defined rotating speed of the screw 8, controlled by control circuit 3, a precise amount of starch 4 is fed into the extruder barrel 17. Control circuit 3 is actuated by speed sensor circuit $I_3$, measuring the rotating speed of the screw 8 and sensing back to a hydraulic proportional flow control valve $O_3$ controlled by control circuit 3, thus assuring a constant rotating speed of the hydraulic motor 10, irrespective of the changing torque resulting from introduction of the starch 4 recharge.

When the load time is completed, the screw 8 rotation is stopped and molding cycle Point D of Fig. 2 is reached. The time from molding cycle Points D to A of Fig. 2 allows for the starch 4 to plasticize completely under controlled temperature conditions as controlled by control circuit 1.

A temperature sensor circuit $I_1$ senses a thyristor heat regulator $O_1$ heating the extruder barrel 17 as directed by control circuit 1.

During the time interval from molding cycle Points B to E on Fig. 2, the mold 6 has cooled down sufficiently so that the finished capsule parts 7 can be ejected from the mold 6.

After ejection of the capsule parts 7, the work cycle returns to Point A of Fig. 2 where a certain volume of plasticized starch 14 has accumulated in front of the screw 8 (sensing circuit $I_4$ is actuated and time has elapsed) so that the work cycle of Fig. 2 can be repeated.

It is important to note the temperature and humidity control loops 5 and 6, for the maintenance of precise water content of the starch 4 in the hopper 5, which is essential for proper operation at the desired speeds.

The microprocessor 28 includes a memory section 51 to store the desired operating parameters; a sensing and signalling section 52 to receive the sensing signals of actual operating conditions, to detect the deviation between the desired and actual operating conditions, and to send signals for adjustment through the actuating section 53 to the thyristors and valves.

Referring now to Fig. 4 there is shown the valve assembly 50 including the outlet port 21, the nozzle 22, the needle valve 23, and the bearing 15. These elements operate as follows:

At Point A in Fig. 2 the needle valve 23 is retracted from the outlet port 21 when there is pressure in the starch 14 while the bearing 15 is pressed against the valve body so as to form an inlet opening 55 for plasticized starch 14 into the nozzle 22 which defines a charging chamber for plasticized starch 14. The plasticized starch 14 is injected through nozzle 22 and into the mold 6 during the mold-filling time between Points A and B in Fig. 2. At Point C in Fig. 2 the needle valve 23 is pushed forward so as to close the outlet port 21 during which time between Point C and E in Fig. 2, the inlet of mold 6 is closed and the capsule part 7 in the mold 6 is cooled. The needle valve 23 remains closed between Point E and A in Fig. 2 during which time the capsule part 7 is ejected from the mold 6.

The one-way valve 15 and the needle valve 23 are actuated by a spring-tensioned lever 25 which normally closes both the outlet port 21 and the nozzle 22 until the lever 25 is cam-actuated pursuant to signals from the microprocessor 28.

The thermomechanical properties of starch, i.e. storage and loss shear modulus at different temperatures, are strongly dependent on its water content The capsule molding process of the present invention can be used for starch with a water content within a range of 5 to 30%. The lower limit is defined by the maximum processing temperature of 240°C, which in turn cannot be exceeded in order to avoid degradation. The upper limit is determined by the stickiness and distortion of the finished capsules. It should also be noted that plasticizing is caused by heat and pressure when dealing with thermoplastic materials. However, with starch, it is also necessary to have strong shearing forces.

The abbreviations in Table 2 below will be used hereinafter in this application:

TABLE 2

| Abbreviations for physical parameters | | |
|---|---|---|
| Abbreviation | Unit | Description |
| $T_a$, $P_a$ | Degree C, $N \times m^{-2}$ | Ambient temperature and pressure. |
| $H(T, P)$ | $KJoule \times Kg^{-2}$ | Enthalpy of starch-water system at a temperature. |
| $K(T, P)$ | $N^{-1} \times m^2$ | Compressibility of the starch at a given temperature and pressure. Its numerical value is the relative volume change due to change of pressure by a unit amount. |
| $(T, P)$ | $(Degree\ C)^{-1}$ | Volumetric thermal expansion coefficient of the starch at a given temperature and pressure. Its numerical value is the relative volume change due to change of temperature by a unit amount. |
| $V(g, T, P)$ | $Kg \times sec^{-1}$ | is the flow rate of the starch at a given temperature and shear deformation rate [sec.$^{-1}$] and pressure. Its numerical value is the volume of a melt leaving the exit cross-sectional area of an injection molding device in unit time due to the applied shear deformation rate. |
| $T_{G1}$; $T_{G2}$ | Deg C | The temperature range of the glass-transition of the starch. |
| $T_{M1}$; $T_{M2}$ | Deg C | The temperature range of the melting of the partially crystalline starch. |
| $T_M$ | | Melting temperature. |
| $T_n(t)$ | Deg C | The temperature of the starch in the nozzle area of the injection unit. |
| $T_t(t)$ | Deg C | The temperature of the starch in the mold. |
| $P_t$ | $N \times m^{-2}$ | The pressure of the starch in the mold. |
| $P_n$ | $N \times m^{-2}$ | The pressure in the nozzle area of the starch. |
| $X$ | | The water content of the starch, expressed as the weight fraction of the water-starch system. |

For the control and regulation of the injection molding process (IMP) we need knowledge of (1) the heat consumption of the melting process:

$$H(T_n, P_n)-H(T_a, P_a)$$

(2) the heating rates of the starch in the injection molding device. To calculate this we need the heat conduction number of the starch and the heat transfer number of the starch and the specific material of construction of the barrel which is in contact with the starch.

The heating rate and the heat consumption of the starch give the minimum time interval necessary to make the starch ready to inject and the necessary heating power of the injection molding device.

(3) $T_n$ depends on X of the starch. If the water content of the starch in the mold is too low, the resulting $T_n$ will be too high and cause degradation. A minimum water content of 5% by weight is required to keep $T_n$ below 240°C.

(4) the flow rate V(g, T, P) is as well strongly dependent on the water content of the starch. To speed up the IMP we need a high flow rate V(g, T, P) which can be achieved by a higher water content.

The upper limit of the water content is defined by the stickiness and mechanical failure of the capsules: a water content of 0.30 cannot be generally exceeded.

The starch in the mold will reduce its volume due to the temperature change $T_t$-$T_a$. This would result in voids and diminution of size of the capsule, which therefore would be of unacceptable quality. It is an important requirement in capsule making that the dimensional deviations are less than 1%. To compensate for shrinking by the temperature change, the mold must be filled at a distinct pressure $P_n$. This filling pressure is determined by the quantities (T, P) and K(T, P). The injection pressure ($P_n$) depends again on $T_n$, which as was shown already is in turn strongly dependent on X.

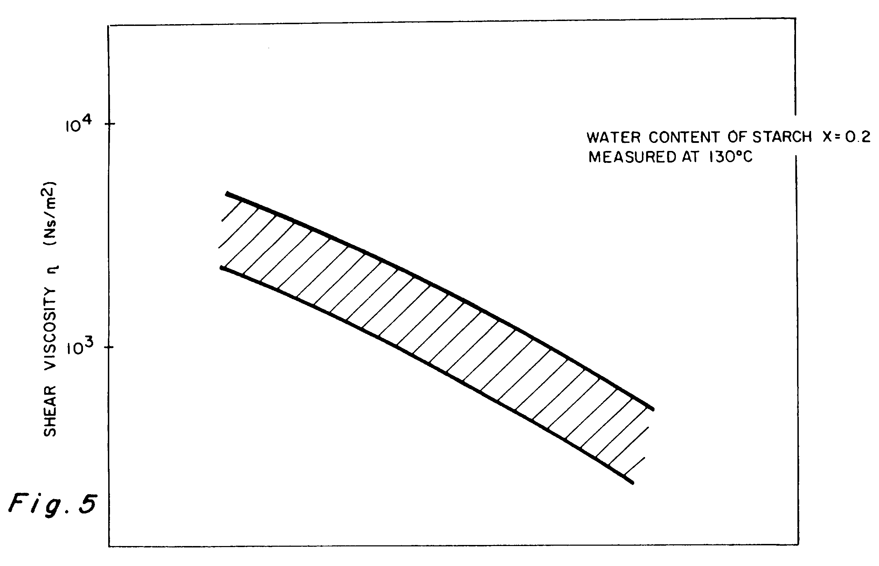

Referring now to Fig. 5, the shear rate dependent shear viscosity of starch at 130 degrees C is shown for starch with a water content X of 0.2.

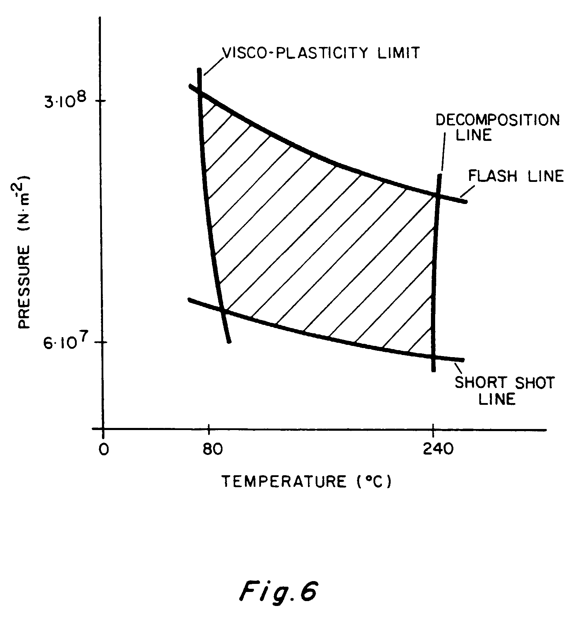

Referring now to Fig. 6, the molding area diagram for starch with water content of 0.24 is shown. During injection molding the plasticized starch is discontinuously extruded and immediately cooled in a mold of the desired shape of the capsule part. Moldability depends on the starch properties and the process conditions, of which the thermomechanical properties of the starch as well as the geometry and the temperature and pressure conditions of the mold are the most important. In the molding area diagram of Fig. 6 the limits of pressure and temperature are indicated for the processing of starch in the combined injection molder-microprocessor of the present invention. The maximum temperature of 240°C is determined by visible degradation of the starch above that limit. The lower temperature limit of 80°C was determined by the development of too high viscosity and melt elasticity in the water content range X=0.05 to 0.30. The higher pressure limit of $3 \times 10^8$ N $\times$ m$^{-2}$ is given by the start of flashing when the melted starch flows in a gap between the various metal dies which make up the molds, thus creating thin webs attached to the molded starch capsule parts at the separating lines. The lower pressure limit of about $6 \times 10^7$ N $\times$ m$^{-2}$ is determined by short shots, when the mold cannot be completely filled by the starch. Shown below in Table 3 are the working parameters for the injection molding process using the starch composition of the present invention.

TABLE 3

| Working parameters for injection molding process | |
|---|---|
| Density | 1.5-$\times 10^3$ kg$\times$m$^{-3}$ |
| Crystallinity | 20 to 70% |
| H($T_n$, $P_n$)-H($T_a$, $P_a$) | 63 KJoule$\times$kg$^{-1}$ |
| Net heating performance for 10 kgs. melt/h (corresponding to $10^6$ capsules/h) | $6.3\times10^2$ KJoule |
| ($T_a$, $P_a$) | $3.1\times10^{-4}$ (Degree° C)$^{-1}$ |
| Contraction due to crystallization | negligible |
| Critical shear deformation rate | $10^4$-$10^6$ sec$^{-1}$ |

The starch compositions of the present invention are extruded and molded as described below:

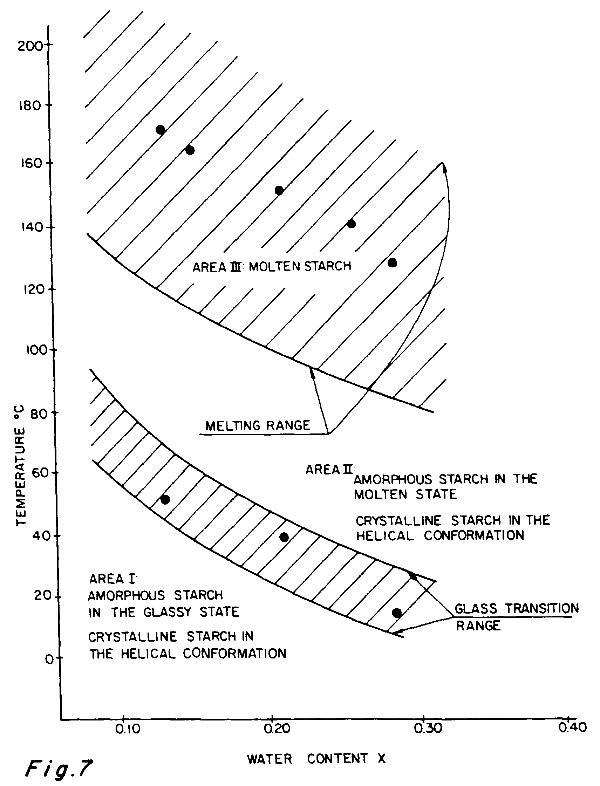

Referring now to Fig. 7 the glass transition range and the melting temperature range is shown as a function of the composition of the starch-water system. The melting range is very broad with over 100°C in comparison with the melting range of e.g. gelatin, which comes to about 20°C. At temperatures below the glass transition range, ordinary starch, as available commercially, is a partially crystalline polymer containing approximately 30-100% amorphous and approximately 0-70% crystalline parts by volume.

By raising the temperature of said starch at a distinct water content the starch passes through the glass transition range.

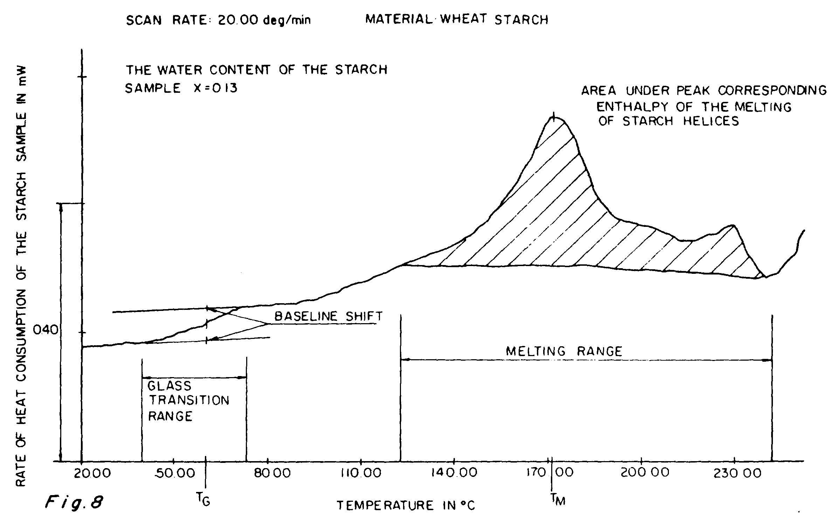

Referring again to Fig. 1, said heating process of the starch will take place within the extruder barrel 17. Referring again to Fig. 2, said heating process of the starch will take place during the entire injection molding work cycle. The area in Fig. 7 between the glass transition range and the melting range is called area II. In area II we find crystalline starch and a starch melt. The glass-transition is not a thermodynamic transition of any order but is characterized by a change of the molecular movement of the starch molecules and by a change of the bulk storage modulus of the amorphous starch by several orders of magnitude. By passing from area II to area I in Fig. 7 the translational movements of the starch molecules or those of large parts of said molecules will be frozen in the glass transition temperature range and this is reflected by a change in the specific heat ($c_p$) and the volumetric thermal expansion coefficient in said temperature range. By passing from area II to area III due to crossing the melting range of the crystalline starch the helically ordered part of the starch will melt. Referring to Fig. 1 said heating process of the starch will take place within the extruder barrel 17. Referring again to Fig. 2, said heating process of the starch will take place during the entire injection molding work cycle. Said helix-coil transition is a true thermodynamic transition of the first order and is an endothermic process. Said transitions can be detected by scanning calorimetry or by measurement of the change of the linear viscoelastic bulk storage modulus due to change of the temperature. A typical plot of a temperature scan with a differential calorimeter is shown in Fig. 8. On the ordinate is plotted the velocity of the heat consumed by the sample relative to a reference (empty sample holder). The velocity of heat consumption of the sample is due to the change of the temperature of the starch sample, and said temperature is plotted on the abscissa as degrees Celsius. The base line shift on said plot is corresponding to the glass transition and the peak to the malting or to the helix-coil transition. The linear viscoelastic bulk storage modulus E can be measured at small sinusoidal, shear deformations of the starch sample.

Referring again to Fig. 1 the heating of the starch 4 to a temperature higher than $T_M$ takes place in the forward part of the extruder barrel 17. Said heating process will be maintained not only by the heating coils 18 but to an important proportion by the internal friction during the screw rotation and the injection process due to the high deformational rates. It was found that the reversible elastic deformation of the injection molded starch 14 after opening the mold 6 is negligible if the temperature of the plasticized starch 14 during the injection process is higher than $T_M$, otherwise the molding sequence would drop by at least an order of magnitude.

Referring again to Fig. 2 the necessary cooling period for the plasticized starch in the molds-to prevent any reversible elastic deformation of said starch-will take place between points B and E of the working cycle. A restriction of the molding sequence to low speed coupling with long keeping of the starch in the mold is undesirable because of two reasons: low output of the product and loss of water content of the starch in the extruder. At the elevated injection temperature there is always a transport of water from the hot to the cold starch in the extruder barrel. Said water transport can be compensated due to the transport of the starch by the screw in the opposite direction.

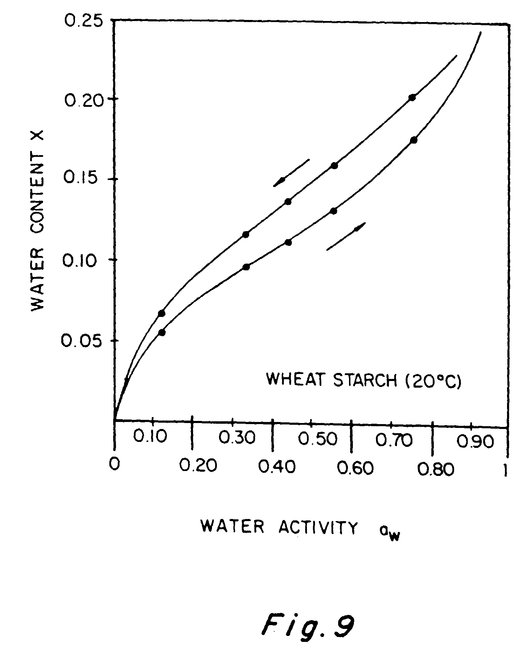

Referring again to Fig. 1 said transport of starch 4 will be maintained by screw 8. Referring again to Fig. 2 said transport of starch will take place between the points C and D of the working cycle. To build up a stationary water content of the starch in the melting area of the extruder barrel, it is necessary to work at an injection sequence which is short. To establish a constant and high enough water content of the starch in the extruder barrel, it is further necessary to use starch with the proper shape of the sorption isotherm. (See Fig. 9). The constant water content of the starch in the extruder barrel is necessary due to the maintenance of constant production conditions. The water content of the starch during the injection must fulfill the condition: X higher than 0.05 otherwise $T_M$ is also higher than 240°C and this is undesirable due to degradation of the starch.

The starch compositions are extruded and injected under the following conditions given in Table 4 below:

**TABLE 4**
**Injection and molding conditions for starch**

| Injection unit | Unit | | | | |
|---|---|---|---|---|---|
| Screw diameter | mm | 18 | 24 | 28 | 32 |
| Injection pressure | $N \times m^{-2}$ | | $2.2 \times 10^8$ | $1.6 \times 10^8$ | $1. \times 10^8$ |
| Calculated injection | $cm^3$ | 21.3 | 38 | 51.7 | 67. |
| Effective screw length | L:D | 18 | 18.8 | 16.1 | 13. |
| Plasticising capacity (PS) 1a) | kg/h (max) | | 13.5 | 21.2 | 21. |
| 11a) | | | 9.2 | 14.5 | 15 |
| 1b) | | | 23.6 | 34 | 36 |
| 11b) | | | 17.5 | 27 | 27. |
| Screw stroke | mm (max.) | 84 | 84 | 84 | 84 |
| Injection capacity | kW | | 30 | 30 | 30 |
| Injection velocity | mm/s (max.) | 2000 | 2000 | 2000 | 2000 |
| Nozzle contact force | kN | 41.2 | 41.2 | 41.2 | 41.2 |
| Screw rotating speed 1a) | $min^{-1}$ | | Var. | 20 | −80 |
| 11a) | | | Var. | 20 | −17 |
| 1b) | | | Var. | 20 | −60 |
| 11b) | | | Var. | 20 | −40 |
| Number of heating zones | | 5 | 5 | 5 | 5 |
| Installed heating capacity | kW | | 6.1 | 6.1 | 6. |
| Molding unit | | | | | |
| Clamping force | kN | | | | 60 |

It has been found that the injection molding process of the present invention can produce quality capsules with various types of starch combined with extenders, for example, vinylacetate; polysaccharides as cellulose, methylcellulose, hydroxypropyl cellulose, hydroxypropyl - methylcellulose hydroxymethylcellulose, hydroxyethylcellulose, sodium carboxy methylcellulose, polyvinyl pyrrolidine, bentonite, agar-agar, gum arabic, guar, dextran, chitin, polymaltose, polyfructose, pectin, alignates or alginic acids; monosaccharides, for example, flucose, fructose or saccharose; oligosaccharises, for example, lactose; silicates, carbonates and bicarbonates. The quantity of extender is controlled so as not to effect the ability of the starch to be injection molded.

In addition it has been found that the injection molding apparatus of the present invention can produce capsules having enteric properties (2 hours resistant in gastric juice, well soluble within 30 minutes in intestinal juice) with various types of starch or with the starches crosslinked during processing as mentioned above with enteric polymers, for example hydroxypropylmethylcellulose phthalate (HPMCP), cellulose, acetylphthalate (CAP), acrylates and methacrylates, polyvinyl - acetate - phthalate (PVAP), phthalated gelatin, succinated gelatin, crotonic acid or shellac. The quantity of extender is controlled so as not to effect the ability of the starch to be injection molded.

For the manufacturing of capsules with different types of starches and/or modified starches and/or extended starchs as mentioned above, the utilization of plasticizers, lubricants and coloring agents specifically of pharmaceutical grades leads to optimal product qualities:

Pharmacologically acceptable plasticizers, such as polyethylene glycol or preferably low-molecular weight organic plasticizers, like glycerol, sorbitol, dioctyl-sodium sulfosuccinate, triethyl citrate, tributyl citrate, 1,2-propylenglycol, mono-, di-, tri-acetates of glycerol etc. are utilized at various concentrations of about 0.5-40% preferably at 0.5-10% based upon the weight of the starch composition.

Pharmacologically acceptable lubricants, such as lipids, i.e. glycerides (oils and fats), wax and phospholipids, such as unsaturated and saturated plant fatty acids and salts thereof, such as the stearates of aluminum, calcium, magnesium and tin; as well as talc, silicones, etc. are to be used at concentrations of about 0.001-10% based upon the weight of the starch composition.

Pharmaceutically acceptable coloring agents, such as azo-dyes and other dyestuffs and pigments as iron oxides, titanium dioxides, natural dyes etc. are used at concentrations of about 0.001-10% preferably at 0.001-5% based upon the weight of the starch composition.

Examples

To test the method and apparatus as described before according to the present invention, batches of commercially available native starch with different water contents and extenders were prepared and conditioned and then tested in an injection molding machine at different working conditions.

Referring to Fig. 2 the cycle times of the injection molding-microprocessor apparatus are as follows:

| Cycle points | Times |
|---|---|
| A-B | 1 second, variable, depending on temperature |
| B-C | 1 second |
| C-D | 1 second |
| D-E | Variable depending on temperature |
| E-A | 1 second |

Pressure in the nozzle: $2 \times 10^8$ N×m$^{-2}$.
Temperatures at different points of screw: (variable, see Examples below).
In the following Examples the abbreviations mean:

$T_b$     temperature at beginning of screw (°C)
$T_m$    temperature at middle of screw (°C)
$T_e$     temperature at end of screw (°C)
$T_n$     temperature at nozzle (°C)
LFV   linear flow velocity (mm/second)
L       flow length (cm)
D      film thickness (cm)

Acceptable starch capsules were processed according to the starch compositions and to the working conditions tabulated in the Examples below:

Example 1

Starch composition:

Wheat starch, water: 79.4% bw, 20.6% bw
Working conditions:

| Number | $T_b$ | $T_m$ | $T_e$ | $T_n$ | L/D | LFV |
|---|---|---|---|---|---|---|
| 305S | 115 | 130 | 140 | 140 | 66 | 820 |

Example 2

Starch composition:

Wheat starch, water, erythrosine: 78.32% bw. 21.6% bw, 0.0078% bw
Working conditions:

| Number | $T_b$ | $T_m$ | $T_e$ | $T_n$ | L/D | LFV |
|--------|-------|-------|-------|-------|-----|------|
| 349S | 110 | 125 | 135 | 135 | 66 | 1000 |

Example 3

Starch composition:

Wheat starch, HPCMP, lubricants + plasticizers, water: 9.2% bw, 74.1% bw, 5.1% bw, 7.5% bw
Working conditions:

| Number | $T_b$ | $T_m$ | $T_e$ | $T_n$ | L/D | LFV |
|--------|-------|-------|-------|-------|-----|------|
| 349S | 110 | 125 | 135 | 135 | 66 | 1000 |

This starch composition yielded an enteric capsule.

Example 4

Starch composition:

Wheat starch, water: 78.5% bw, 21.5% bw
Working conditions:

| Number | $T_b$ | $T_m$ | $T_e$ | $T_n$ | L/D | LFV |
|--------|-------|-------|-------|-------|-----|-----|
| 400S | 130 | 150 | 160 | 160 | 66 | 820 |
| 404S | 110 | 115 | 125 | 125 | 66 | 820 |

Example 5

Starch composition:

Wheat starch, water, 87.3% bw, 12.7% bw
Working conditions:

| Number | $T_b$ | $T_m$ | $T_e$ | $T_n$ | L/D | LFV |
|--------|-------|-------|-------|-------|-----|-----|
| 405S | 150 | 160 | 170 | 170 | 66 | 820 |

Example 6

Starch composition:

Wheat starch, calcium-stearate, water: 76.8% bw, 3% bw, 20.2% bw
Working conditions:

| Number | $T_b$ | $T_m$ | $T_e$ | $T_n$ | L/D | LFV |
|--------|-------|-------|-------|-------|-----|-----|
| 411S | 100 | 110 | 135 | 135 | 66 | 880 |
| 413S | 130 | 140 | 160 | 160 | 66 | 820 |

Example 7

Starch composition:

Wheat starch, glycerin, water: 77.2% bw, 3% bw, 19.8% bw
Working conditions:

| Number | $T_b$ | $T_m$ | $T_e$ | $T_n$ | L/D | LFV |
|--------|-------|-------|-------|-------|-----|-----|
| 410S | 100 | 110 | 130 | 130 | 66 | 860 |
| 414S | 130 | 140 | 160 | 160 | 66 | 840 |

Example 8

Starch composition:

Wheat starch, polyethylene-glycol (10,000 m.w.), water, talcum: 72.5% bw, 3% bw, 22.5% bw, 2% bw
Working conditions:

| Number | $T_b$ | $T_m$ | $T_e$ | $T_n$ | L/D | LFV |
|--------|-------|-------|-------|-------|-----|-----|
| 412S | 100 | 110 | 130 | 130 | 66 | 840 |
| 415S | 130 | 140 | 160 | 160 | 66 | 840 |

Example 9

Starch composition:

Potato starch, water: 80.7% bw. 19.3% bw
Working conditions:

| Number | $T_b$ | $T_m$ | $T_e$ | $T_n$ | L/D | LFV |
|--------|-------|-------|-------|-------|-----|-----|
| 417S | 100 | 110 | 130 | 130 | 66 | 840 |

Example 10

This example demonstrated the dependence of the capsules, disintegration properties on the content of amylose. For these tests, the capsules were filled with lactose.

| starch composition | working conditions (°C) $T_b$, $T_m$, $T_e$, $T_n$, L/D, LFV | disintegration property of the capsules |
|--------------------|-------------------------------------------------------------|-----------------------------------------|
| maize starch (about 20% amylose) | 110, 120, 140, 140, 66, 840 | floculation in water of 36°C, disintegration within 30 minutes |
| maize starch (65% amylose) 80% b. w., water 20% b.w. | 110, 120, 140, 140, 66, 840 | no opening in water of 36°C within 30 minutes |

(continued)

| maize starch (0% amylose, 100% amylopectin) 79.2 b.w., water 20.8% b.w. | 110, 120, 140, 140, 66, 836 | disintegration in water of 36°C, disintegration within 30 minutes |
|---|---|---|

**Claims**

1. A process for forming starch into an article using an injection molding technique, which process comprises:

   (a) maintaining under controlled conditions of temperature and pressure a composition comprising starch which is not chemically modified and which does not contain protein material or alkali metal or alkaline earth metal salt of protein; and water at a water content in the range of from 5 to 30% by weight based on the weight of the composition;
   (b) heating the composition, while maintaining said water content, at elevated pressure and under shear in a closed volume to a temperature in the range of from 80 to 240°C, above its glass transition temperature and melting point thereby to form a melt;
   (c) further heating the melt at elevated pressure and under shear in the closed volume to a temperature in the range of from 80 to 240°C for a time sufficient to dissolve and plasticize the melt into molecularly dispersed form;
   (d) injecting the plasticized melt into a mold cavity while maintaining the water content of the melt in said range;
   (e) cooling the melt in the mold to a temperature below its glass transition temperature, thereby to form a molded article in the mold; and
   (f) ejecting the molded article from the mold cavity.

2. A process according to claim 1, wherein elevated pressure in step (d) is in the range of from $600 \times 10^5$ to $3,000 \times 10^5$ Newton/square meter.

3. A process according to claim 1 or claim 2, wherein the starch on the whole, or partially, is a corn, wheat, potato, rice or tapioca starch or a mixture thereof.

4. A process according to any preceding claim, wherein the composition further includes one or more extenders and/or one or more plasticizers and/or one or more lubricants and/or one or more coloring agents.

5. A process according to claim 4, wherein the plasticizer is present in an amount of from 0.5 to 40% based upon the weight of the composition.

6. A process according to claim 4 or claim 5, wherein the lubricant is present in an amount of 0.001 to 10% based on the weight of the composition.

7. A process according to any one of claims 4 to 6, wherein the coloring agent is present in an amount of from 0.001% to 10% based on the weight of the composition.

8. A process according to any one of claims 4 to 7, wherein the extender is chosen from vinylacetate, polysaccharides such as cellulose, methylcellulose, hydroxypropylcellulose, hydroxypropylmethylcellulose, hydroxymethylcellulose, hydroxyethylcellulose, sodium carboxymethylcellulose, polyvinylpyrrolidone, agar-agar, gum arabic, guar, dextran, chitin, polymaltose, polyfructose, pectin, alginates, alginic acids, monosaccharides, preferably glucose, fructose or saccharose, and oligosaccharides, preferably lactose, bentonite, silicates, carbonates and bicarbonates.

9. A process according to any one of claims 4 to 8, wherein the plasticizer is chosen from: polyethylene glycol and low molecular weight organic plasticizers, including glycerol, sorbitol, dioctyl-sodium sulphosuccinate, triethyl citrate, tributyl citrate, 1,2-propyleneglycol, mono- di-, and tri-acetates of glycerol.

10. A process according to any one of claims 4 to 9, wherein the lubricant is chosen from: lipids, unsaturated and saturated plant fatty acids and salts thereof; stearates of aluminium, calcium, magnesium and tin; talc and silicones.

11. A process according to claim 10, wherein the lubricant is a glyceride, a phospholipid or a mixture thereof in an amount of from 0.001 to 10% by weight of the composition.

12. A process according to any one of claims 1 to 11, wherein the starch is mixed with one or more polymer having enteric properties and being chosen from: hydroxypropylmethylcellulose phthalate (HPMCP), celluloseacetyl phthalate (CAP), acrylates and methacrylates, polyvinylacetate phthalate (PVAP), phthalated gelatin, succinated gelatin, crotonic acid, and shellac.

13. An injection molded article whenever produced by a process as claimed in any one of Claims 1 to 12.

14. A capsule whenever produced by a process as claimed in any one of claims 1 to 12.

15. A product formed by a process as claimed in any one of claims 1 to 12, wherein the product may be:

a candy, a packaging container for packaging, for example, foodstuffs, pharmaceuticals, chemicals, dyestuffs, spices, fertilizing combinations, seeds, cosmetics and agricultural products;
matrices of various shapes and sizes which may comprise substances and/or active ingredients (for example, foodstuffs, pharmaceuticals, chemicals, dyestuffs, spices, fertilizing combination, seeds, cosmetics and agricultural products) which are microdispersed within the matrix and released from it through disintegration and/or dissolution and/or bioerosion and/or diffusion depending on the solubility characteristics of the starch composition used, and sometimes resulting in a controlled release delivery system for the microdispersed substance; and
medical and surgery products.

16. A pharmaceutical, whenever microdispersed within a starch matrix as described in claim 15.

17. The use of a composition comprising: starch which is not chemically modified and which does not contain protein material or alkali metal or alkaline earth metal salt of protein; and water, which composition has a water content in the range of from 5 to 30% by weight of the composition, in an injection molding process as claimed in any one of claims 1 to 12.

18. A composition in the form of a molecular dispersion of starch and water at an elevated temperature, characterised in that it is obtained by:

(a) maintaining under controlled conditions of temperature and pressure a composition comprising: starch which is not chemically modified and which does not contain protein material or alkali metal or alkaline earth metal salt of protein; and water, at a water content in the range of from 5 to 30% by weight based on the weight of the composition;
(b) heating the composition, while maintaining said water content, at elevated pressure and under shear in a closed volume to a temperature in the range of from 80 to 240°C, above its glass transition temperature and melting point thereby to form a melt; and
(c) further heating the melt at elevated pressure and under shear in the closed volume to a temperature in the range of from 80 to 240°C for sufficient time to dissolve and plasticize the melt into molecularly dispersed form.

19. A process for preparing a composition in the form of a molecular dispersion of starch and water at an elevated temperature, which process comprises the steps of:

(a) maintaining under controlled conditions of temperature and pressure a composition comprising: starch which is not chemically modified and which does not contain protein material or alkali metal or alkaline earth metal salt of protein; and water, at a water content in the range of from 5 to 30% by weight based on the weight of the composition;
(b) heating the composition, while maintaining said water content, at elevated pressure and under shear in a closed volume to a temperature in the range of from 80 to 240°C, above its glass transition temperature and melting point thereby to form a melt; and
(c) further heating the melt at elevated pressure and under shear in the closed volume to a temperature in the range of from 80 to 240°C for a time sufficient to dissolve and plasticize the melt into molecularly dispersed form.

**20.** A molded article made from a starch/water composition as described in claim 18, wherein the wall structure of said article is essentially amorphous.

**Patentansprüche**

**1.** Verfahren zur Formung einer Stärke zu einem Gegenstand unter Verwendung einer Spritzgußtechnik, welches Verfahren umfaßt:

(a) das Halten einer Zusammensetzung umfassend eine Stärke, die nicht chemisch modifiziert ist und die kein Proteinmaterial oder Alkalimetall- oder Erdalkalimetallsalz eines Proteins enthält, und Wasser bei einem Wassergehalt im Bereich von 5 bis 30 Gew.-%, bezogen auf das Gewicht der Zusammensetzung, unter gesteuerten Temperatur- und Druckbedingungen;

(b) Erhitzen der Zusammensetzung unter Beibehaltung des Wassergehalts bei erhöhtem Druck und unter Scherung in einem geschlossenen Volumen auf eine Temperatur im Bereich von 80 bis 240°C, über ihrer Glasumwandlungstemperatur und ihrem Schmelzpunkt zur Bildung einer Schmelze;

(c) weiteres Erhitzen der Schmelze bei erhöhtem Druck und unter Scherung im geschlossenen Volumen auf eine Temperatur im Bereich von 80 bis 240°C während einer Zeit, die zum Lösen und Plastifizieren der Schmelze in molekular dispergierte Form ausreicht;

(d) Spritzen der plastifizierten Schmelze in einen Formhohlraum, während der Wassergehalt der Schmelze in diesem Bereich gehalten wird;

(e) Kühlen der Schmelze in der Form auf eine Temperatur unter ihrer Glasumwandlungstemperatur zur Bildung eines geformten Gegenstandes in der Form; und

(f) Auswerfen des geformten Gegenstandes aus dem Formhohlraum.

**2.** Verfahren nach Anspruch 1, worin der erhöhte Druck in Schritt (d) im Bereich von $600 \times 10^5$ bis $3000 \times 10^5$ Newton/Quadratmeter liegt.

**3.** Verfahren nach Anspruch 1 oder Anspruch 2, worin die Stärke insgesamt oder teilweise eine Mais-, Weizen-, Kartoffel-, Reis-, Tapiokastärke oder eine Mischung davon ist.

**4.** Verfahren nach einem der vorhergehenden Ansprüche, worin die Zusammensetzung weiter ein oder mehrere Streckmittel und/oder einen oder mehrere Plastifikatoren und/oder ein oder mehrere Gleitmittel und/oder einen oder mehrere Farbstoffe enthält.

**5.** Verfahren nach Anspruch 4, worin der Plastifikator in einer Menge von 0,5 bis 40%, bezogen auf das Gewicht der Zusammensetzung, vorhanden ist.

**6.** Verfahren nach Anspruch 4 oder Anspruch 5, worin das Gleitmittel in einer Menge von 0,001 bis 10%, bezogen auf das Gewicht der Zusammensetzung, vorhanden ist.

**7.** Verfahren nach einem der Ansprüche 4 bis 6, worin der Farbstoff in einer Menge von 0,001% bis 10%, bezogen auf das Gewicht der Zusammensetzung, vorhanden ist.

**8.** Verfahren nach einem der Ansprüche 4 bis 7, worin das Streckmittel ausgewählt ist aus Vinylacetat, Polysacchariden, wie Cellulose, Methylcellulose, Hydroxypropylcellulose, Hydroxypropylmethylcellulose, Hydroxymethylcellulose, Hydroxyethylcellulose, Natriumcarboxymethylcellulose, Polyvinylpyrrolidon, Agar-Agar, Gummi arabicum, Guar, Dextran, Chitin, Polymaltose, Polyfructose, Pektin, Alginaten, Alginsäuren, Monosacchariden, vorzugsweise Glukose, Fruktose, oder Saccharose, und Oligosacchariden, vorzugsweise Laktose, Bentonit, Silikaten, Carbonaten und Bicarbonaten.

**9.** Verfahren nach einem der Ansprüche 4 bis 8, worin der Plastifikator ausgewählt ist aus: Polyethylenglykol und niedrigmolekulargewichtigen organischen Plastifikatoren, inklusive Glycerin, Sorbit, Dioctylnatriumsulfosuccinat, Triethylcitrat, Tributylcitrat, 1,2-Propylenglykol, Mono-, Di- und Triacetaten von Glycerin.

**10.** Verfahren nach einem der Ansprüche 4 bis 9, worin das Gleitmittel ausgewählt ist aus: Lipiden, ungesättigten und gesättigten Pflanzenfettsäuren und Salzen davon; Stearaten von Aluminium, Calcium, Magnesium und Zinn; Talkum und Silikonen.

11. Verfahren nach Anspruch 10, worin das Gleitmittel ein Glycerid, ein Phospholipid oder eine Mischung davon in einer Menge von 0,001 bis 10 Gew.-% der Zusammensetzung ist.

12. Verfahren nach einem der Ansprüche 1 bis 11, worin die Stärke mit einem oder mehreren Polymeren mit enterischen Eigenschaften und ausgewählt aus: Hydroxypropylmethylcellulosephthalat (HPMCP), Celluloseacetylphthalat (CAP), Acrylaten und Methacrylaten, Polyvinylacetatphthalat (PVAP), Phthalat-Gelatine, Succinat-Gelatine, Krotonsäure und Schellack gemischt wird.

13. Spritzgußartikel, wenn hergestellt mittels eines Verfahrens, wie in einem der Ansprüche 1 bis 12 beansprucht.

14. Kapsel, wenn hergestellt mittels eines Verfahrens, wie in einem der Ansprüche 1 bis 12 beansprucht.

15. Produkt, gebildet mittels eines Verfahrens, wie in einem der Ansprüche 1 bis 12 beansprucht, worin das Produkt sein kann:

eine Süßigkeit, ein Verpackungsbehälter zum Verpacken von beispielsweise Nahrungsmitteln, Pharmazeutika, Chemikalien, Farbstoffen, Gewürzen, Düngemittelkombinationen, Samen, Kosmetika und landwirtschaftlichen Produkten;
Matrizen verschiedener Formen und Größen, die Substanzen und/oder aktive Ingredienzien (beispielsweise Nahrungsmittel, Pharmazeutika, Chemikalien, Farbstoffe, Gewürze, Düngemittelkombinationen, Samen, Kosmetika und landwirtschaftliche Produkte) umfassen können, die innerhalb der Matrix mikrodispergiert sind und aus dieser durch Zerfall und/oder Auflösen und/oder Bioerosion und/oder Diffusion, je nach den Löslichkeitseigenschaften der verwendeten Stärkezusammensetzung, freigesetzt werden, was manchmal zu einem gesteuerten Abgabe-Zufuhr-System für die mikrodispergierte Substanz führt; und
medizinische und chirurgische Produkte.

16. Pharmazeutisches Mittel, wenn dieses innerhalb einer Stärkematrix, wie in Anspruch 15 beschrieben, mikrodispergiert ist.

17. Verwendung einer Zusammensetzung umfassend eine Stärke, die nicht chemisch modifiziert ist und kein Proteinmaterial oder Alkalimetall- oder Erdalkalimetallsalz eines Proteins enthält; und Wasser, wobei die Zusammensetzung einen Wassergehalt im Bereich von 5 bis 30 Gew.-% der Zusammensetzung aufweist, bei einem Spritzgußverfahren, wie in einem der Ansprüche 1 bis 12 beansprucht.

18. Zusammensetzung in Form einer molekularen Dispersion von Stärke und Wasser bei einer erhöhten Temperatur, dadurch gekennzeichnet, daß sie erhalten wird durch:

(a) Halten einer Zusammensetzung, die Stärke, die nicht chemisch modifiziert ist und die kein Proteinmaterial oder Alkalimetall- oder Erdalkalimetallsalz eines Proteins enthält, und Wasser bei einem Wassergehalt im Bereich von 5 bis 30 Gew.-%, bezogen auf das Gewicht der Zusammensetzung, umfaßt, unter gesteuerten Temperatur- und Druckbedingungen;
(b) Erhitzen der Zusammensetzung unter Beibehaltung des Wassergehalts unter erhöhtem Druck und unter Scherung in einem geschlossenen Volumen auf eine Temperatur im Bereich von 80 bis 240°C, über ihrer Glasumwandlungstemperatur und ihrem Schmelzpunkt, um dadurch eine Schmelze zu bilden; und
(c) weiteres Erhitzen der Schmelze unter erhöhtem Druck und unter Scherung im geschlossenen Volumen auf eine Temperatur im Bereich von 80 bis 240°C während einer Zeit, die zum Lösen und Plastifizieren der Schmelze in molekular dispergierte Form ausreicht.

19. Verfahren zur Herstellung einer Zusammensetzung in Form einer molekularen Dispersion von Stärke und Wasser bei erhöhter Temperatur, welches Verfahren die Schritte umfaßt:

(a) Halten einer Zusammensetzung, die Stärke, die nicht chemisch modifiziert ist und die kein Proteinmaterial oder Alkalimetall- oder Erdalkalimetallsalz eines Proteins enthält, und Wasser bei einem Wassergehalt im Bereich von 5 bis 30 Gew.-%, bezogen auf das Gewicht der Zusammensetzung, umfaßt, unter gesteuerten Temperatur- und Druckbedingungen;
(b) Erhitzen der Zusammensetzung, unter Beibehaltung des Wassergehalts, unter erhöhtem Druck und unter Scherung in einem geschlossenen Volumen auf eine Temperatur im Bereich von 80 bis 240°C, über ihrer Glasumwandlungstemperatur und ihrem Schmelzpunkt, um dadurch eine Schmelze zu bilden; und

EP 0 118 240 B2

(c) weiteres Erhitzen der Schmelze bei erhöhtem Druck und unter Scherung im geschlossenen Volumen auf eine Temperatur im Bereich von 80 bis 240°C während einer Zeit, die zum Lösen und Plastifizieren der Schmelze in molekular dispergierte Form ausreicht.

**20.** Geformter Gegenstand aus einer Stärke/WasserZusammensetzung, wie in Anspruch 18 beschrieben, wobei die Wandstruktur dieses Gegenstandes im wesentlichen amorph ist.

**Revendications**

1. Un procédé de conformation d'un amidon en un article en utilisant une technique de moulage par injection, ce procédé consistant en:

(a) le maintien dans des conditions contrôlées de température et de pression d'une composition comprenant un amidon, qui n'est pas chimiquement modifié et qui ne contient pas de substances protéiques ni de sel de métal alcalin ou alcalino-terreux de protéines et de l'eau, à une teneur en eau comprise dans la fourchette de 5 et 30% en poids par rapport au poids de la composition;
(b) le chauffage la composition, tout en respectant ladite teneur en eau, sous pression élevée et sous cisaillement dans une enceinte fermée, à une température de 80 à 240°C, au-dessus de sa température de transition vitreuse et de son point de fusion pour former une masse fondue;
(c) la poursuite du chauffage de la masse fondue sous pression élevée et sous cisaillement dans l'enceinte fermée, à une température de 80 à 240°C, pendant une durée suffisante pour dissoudre et plastifier sa masse fondue en une forme moléculairement dispersée;
(d) l'injection de la masse fondue et plastifiée dans la cavité d'un moule tout en maintenant la teneur en eau de cette masse fondue dans ladite fourchette;
(e) le refroidissement de la masse fondue dans le moule à une température inférieure à sa température de transition vitreuse pour former ainsi, dans le moule, un article moulé; et
(f) l'éjection de l'article moulé de la cavité du moule.

2. Un procédé selon la revendication 1, dans lequel la pression élevée dans l'étape (c) est comprise entre 600 x 10$^5$ et 3000 x 10$^5$ N/m$^2$.

3. Un procédé selon l'une quelconque des revendications 1 ou 2, dans lequel l'amidon est totalement ou partiellement un amidon de maïs, de blé, de pomme de terre, de riz ou de tapioca, ou un mélange de ceux-ci.

4. Un procédé selon l'une quelconque des revendications précédentes, dans lequel la composition comprend encore un ou plusieurs agents diluants et/ou un ou plusieurs plastifiants et/ou un ou plusieurs lubrifiants et/ou un ou plusieurs agents colorants.

5. Un procédé selon la revendication 4, dans lequel le plastifiant est présent en quantité comprise entre 0,5 et 40% par rapport au poids de la composition.

6. Un procédé selon la revendication 4 ou 5, dans lequel le lubrifiant est présent en quantité de 0,001 à 10% par rapport au poids de la composition.

7. Un procédé selon une quelconque des revendications 4 à 6, dans lequel l'agent colorant est présent en quantité comprise entre 0.001 et 10% par rapport au poids de la composition.

8. Un procédé selon une quelconque des revendications 4 à 7, dans lequel l'agent diluant est choisi parmi les suivants: acétate de vinyle, polysaccharides tels que cellulose, méthylcellulose, hydroxypropylcellulose, hydroxypropylméthylcellulose, hydroxyméthylcellulose, hydroxyéthylcellulose, carboxyméthylcellulose de sodium, polyvinylpyrrolidone, agar-agar, gomme arabique, gomme de guar, dextrane, chitine, polymaltose, polyfructose, pectine, alginates, acides alginiques, monosaccharides, de préférence glucose, fructose ou saccharose; et oligosaccharides, de préférence lactose; bentonite, silicates, carbonates et bicarbonates.

9. Un procédé selon l'une quelconque des revendications 4 à 8, dans lequel le plastifiant est choisi parmi les suivants: polyéthylèneglycol et plastifiants organiques de faible poids moléculaire, dont glycérol, sorbitol, dioctyl sulfosuccinate de sodium, citrate de triéthyle, citrate de tributyle, 1,2-propylèneglycol, mono-, di- et tri-acétates de glycérol.

18

**10.** Un procédé selon l'une quelconque des revendications 4 à 9, dans lequel le lubrifiant est choisi parmi les suivants: lipides, acides gras végétaux saturés et insaturés et leurs sels; stéarates d'aluminium, de calcium, de magnésium et d'étain, talc et silicones.

**11.** Un procédé selon la revendication 10, dans lequel le lubrifiant est un glycéride, un phospholipide ou un de leurs mélanges, en quantité comprise entre 0,001 et 10% en poids par rapport au poids de la composition.

**12.** Un procédé selon l'une quelconque des revendications 1 à 11, dans lequel l'amidon est mélangé avec un ou plusieurs polymères présentant des propriétés entériques choisies parmi les suivants: hydroxypropylméthylcellulose phtalate (HPMCP), acétylphtalate de cellulose (CAP), acrylates et méthacrylates, polyvinylacétate phtalate (PVAP), gélatine phtalatée. gélatine succinatée, acide crotonique et shellac.

**13.** Un article moulé par injection produit chaque fois par un procédé selon l'une quelconque des revendications 1 à 12.

**14.** Une gélule produite par un procédé selon l'une quelconque des revendications 1 à 12.

**15.** Un produit fabriqué par un procédé selon l'une quelconque des revendications 1 à 12, dans lequel le produit peut être:

- une confiserie, un récipient d'emballage pour emballage, par exemple d'aliments, produits pharmaceutiques, produits chimiques, des colorants, des épices, des associations d'engrais, des graines. des produits cosmétiques et agricoles;
- des matrices de différentes formes et dimensions pouvant comprendre des substances et/ou des ingrédients actifs (par exemple produits alimentaires, produits pharmaceutiques, produits chimiques, colorants, épices, associations d'engrais, graines, produits cosmétiques et agricoles) microdispersés au sein de la matrice et libérés de celle-ci par désintégration et/ou par dissolution et/ou par bio-érosion et/ou par diffusion selon les caractéristiques de solubilité de la composition d'amidon utilisée. et conduisant quelquefois à un système de libération prolongée de la substance microdispersée; et
- de produits médicaux et de produits chirurgicaux.

**16.** Un produit pharmaceutique, lorsqu'il est microdispersé dans une matrice d'amidon telle que décrite dans la revendication 15.

**17.** Utilisation d'une composition contenant de l'amidon qui n'est pas chimiquement modifié et qui ne contient pas d'addition de protéine ni de sel de métal alcalin ou alcalino-terreux de protéines, et présentant une teneur en eau comprise entre 5 et 30% par rapport au poids de la composition dans un procédé de moulage par injection selon l'une quelconque des revendications 1 à 12.

**18.** Une composition sous la forme d'une dispersion moléculaire d'amidon et d'eau à une température élevée, caractérisée en ce qu'elle est obtenue par:

(a) le maintien dans des conditions contrôlées de température et de pression d'une composition comprenant un amidon, qui n'est pas chimiquement modifié et qui ne contient pas de substances protéiques ni de sel de métal alcalin ou alcalino-terreux de protéines et de l'eau, à une teneur en eau comprise dans la fourchette de 5 et 30% en poids par rapport au poids de la composition;
(b) le chauffage la composition, tout en respectant ladite teneur en eau, sous pression élevée et sous cisaillement dans une enceinte fermée, à une température de 80 à 240°C, au-dessus de sa température de transition vitreuse et de son point de fusion pour former une masse fondue;
(c) la poursuite du chauffage de la masse fondue sous pression élevée et sous cisailement dans l'enceinte fermée, à une température de 80 à 240°C, pendant une durée suffisante pour dissoudre et plastifier sa masse fondue en une forme moléculairement dispersée;

**19.** Un procédé de préparation d'une composition sous la forme d'une dispersion moléculaire d'amidon et d'eau à température élevée, ce procédé comprenant les étapes suivantes:

(a) le maintien dans des conditions contrôlées de température et de pression d'une composition comprenant un amidon, qui n'est pas chimiquement modifié et qui ne contient pas de substances protéiques ni de sel de métal alcalin ou alcalino-terreux de protéines et de l'eau, à une teneur en eau comprise dans la fourchette de 5 et 30% en poids par rapport au poids de la composition;

(b) le chauffage la composition, tout en respectant ladite teneur en eau, sous pression élevée et sous cisaillement dans une enceinte fermée, à une température de 80 à 240°C, au-dessus de sa température de transition vitreuse et de son point de fusion pour former une masse fondue;

(c) la poursuite du chauffage de la masse fondue sous pression élevée et sous cisaillement dans l'enceinte fermée, à une température de 80 à 240°C, pendant une durée suffisante pour dissoudre et plastifier sa masse fondue en une forme moléculairement dispersée;

20. Un article moulé fabriqué à partir d'une composition amidon/eau telle que décrite dans la revendication 18, dans lequel la structure de la paroi de cet article est pratiquement amorphe.

Fig. 1

Fig. 2

Fig.4

*Fig. 3*

Fig. 5

SHEAR VISCOSITY η (Ns/m²)

$10^4$

$10^3$

WATER CONTENT OF STARCH X= O.2
MEASURED AT 130°C

EP 0 118 240 B2

Fig.6

25

*Fig.7*

SCAN RATE: 20.00 deg/min          MATERIAL WHEAT STARCH

THE WATER CONTENT OF THE STARCH
SAMPLE X = 0.13

AREA UNDER PEAK CORRESPONDING
ENTHALPY OF THE MELTING
OF STARCH HELICES

RATE OF HEAT CONSUMPTION OF THE STARCH SAMPLE IN mW

BASELINE SHIFT

0.40

MELTING RANGE

GLASS
TRANSITION
RANGE

2000          5000          8000          11000          14000          170 00          200 00          230 00

$T_G$          TEMPERATURE IN °C          $T_M$

Fig. 8

EP 0 118 240 B2

Fig.9